(19) Europäisches Patentamt
European Patent Office
Office européen des brevets

(11) Publication number: **0 049 155**
**B1**

(12) # EUROPEAN PATENT SPECIFICATION

(45) Date of publication of patent specification: **12.02.86**

(51) Int. Cl.⁴: **C 08 G 81/02,** C 08 F 8/42, C 08 G 77/04, H 01 B 3/44

(21) Application number: **81304496.3**

(22) Date of filing: **29.09.81**

(54) **Production of silane modified copolymers of alkylene-alkyl acrylates, polysiloxanes having combined therein organo titanates used in their production, process for producing such polysiloxanes, and electrical conductors insulated by cured silane modified copolymers.**

(30) Priority: **30.09.80 US 192319**

(43) Date of publication of application:
**07.04.82 Bulletin 82/14**

(45) Publication of the grant of the patent:
**12.02.86 Bulletin 86/07**

(84) Designated Contracting States:
**DE FR GB IT NL SE**

(56) References cited:
**EP-A-0 004 752**
**EP-A-0 016 663**
**DE-B-1 495 860**
**DE-B-2 410 990**

(73) Proprietor: **UNION CARBIDE CORPORATION**
**39 Old Ridgebury Road**
**Danbury Connecticut 06817 (US)**

(72) Inventor: **Keogh, Michael John**
**13 Jeffrey Lane**
**Bridgewater New Jersey (US)**

(74) Representative: **Baverstock, Michael George Douglas et al**
**BOULT, WADE & TENNANT 27 Furnival Street**
**London, EC4A 1PQ (GB)**

**The file contains technical information submitted after the application was filed and not included in this specification**

**EP 0 049 155 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This application relates to the production of silane modified copolymers of alkylene-alkyl acrylates, by the reaction of a polysiloxane and an alkylene-alkyl acrylate copolymer, which can be extruded about electrical conductors and water-cured, if so desired, to crosslinked products, providing insulation about electrical conductors which is characterized by improved surface characteristics. More particularly, this invention relates to polysiloxanes having combined therein an organo titanate and the use thereof in the production of silane modified copolymers of alkylene-alkyl acrylates.

Water-curable, silane modified copolymers of alkylene-alkyl acrylates and a process for the preparation thereof by reacting a mixture containing a silane and an alkylene-alkyl acrylate copolymer are described in detail in European Patent Serial No 4752. The Silane modified copolymers, as described in that patent, can be extruded about electrical conductors such as wire and cable and water-cured to crosslinked products to provide insulation thereon of excellent quality.

It is customary, prior to extruding silane modified alkylene-alkyl acrylate copolymers about wire and cable, to ensure removal therefrom of undesirable volatiles. The presence of undesirable volatiles could lead to the formation of voids in the extruded insulation, marring the appearance of the final insulated product and, in some instances, shortening its working life. In addition, removal of undesirable volatiles from the silane modified copolymers reduces odor problems at the extruder and in the insulated wire or cable. Obviously, removal of volatiles from the silane modified copolymers, by a subsequent devolatilization step after the preparation of the copolymers, increases the time required to produce an insulated product and, also, increases the overall cost thereof.

The present invention provides for the production of silane modified copolymers of alkylene-alkyl acrylates which are free of undesirable volatiles and consequently need not be subjected to a subsequent devolatilization step. The silane modified copolymers, prepared in accordance with the present invention, can be directly extruded about wires and cables and water-cured to crosslinked products to provide insulation thereon, free of undesirable voids and odors.

In one aspect of the present invention a silane modified copolymer is produced by reacting a mixture containing an alkylene-alkyl acrylate copolymer and a polysiloxane containing repeating units of the formula:

Formula I 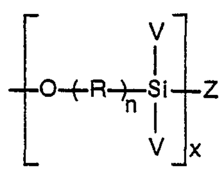

wherein R is a hydrocarbon radical; each V, which can be the same or different, is hydrogen, a hydrocarbon radical or a hydrolyzable group; Z is a hydrolyzable group; n is an integer having a value of one to 18 inclusive and x is an integer having a value of at least 2, generally 2 to 1000 inclusive, preferably 5 to 25 inclusive.

Illustrative of suitable hydrocarbon radicals for R are alkylene radicals having one to 18 carbon atoms inclusive, preferably one to 6 carbon atoms inclusive, such as methylene, ethylene, propylene, butylene, and hexylene.

As stated, each V can be hydrogen, a hydrocarbon radical or a hydrolyzable group. Illustrative of suitable hydrocarbon radicals are alkyl radicals having one to 18 carbon radicals, preferably one to 6 carbon atoms inclusive such as methyl, ethyl, *n*-propyl, isopropyl, *n*-butyl, and *n*-hexyl; alkoxy radicals having one to 18 carbon atoms inclusive, preferably one to 6 carbon atoms inclusive, such as methoxy, ethoxy, propoxy, hexoxy, dodecyloxy, and methoxyethoxy; aryl radicals having 6 to 8 carbon atoms inclusive such as phenyl, methyl phenyl, and ethyl phenyl; and cycloaliphatic radicals having 5 to 8 carbon atoms inclusive such as cyclopentyl, cyclohexyl, and cyclohexyloxy.

Z, as previously stated, is a hydrolyzable group among which can be noted alkoxy radicals having one to 18 carbon atoms inclusive, preferably one to 6 carbon atoms inclusive such as methyloxymethyl, methyloxypropyl, ethyloxyethyl, ethyloxypropyl, propyloxypropyl, propyloxybutyl and propyloxyhexyl; oxy aryl radicals such as oxyphenyl; oxyaliphatic radicals such as oxyhexyl; halogens such as chlorine, and other hydrolyzable groups as further described in U.S.—A—3,408,420 to John B. Wiggill patented October 29, 1968.

Polysiloxanes having repeating units falling within the scope of Formula I can be prepared as described in U.S.—A—3,193,567 to Gerd Rossmy patented July 6, 1965 or by condensing and polymerizing a silane falling within the scope of Formula II in the presence of a metal carboxylate. Among suitable metal carboxylates can be noted dibutyltin dilaurate, stannous acetate, stannous octoate, lead naphthenate, zinc octoate, and iron 2-ethyl hexoate. Conditions employed for the production of polysiloxanes, reaction temperatures, amount of materials, etc, using metal carboxylates as catalysts are the same as subsequently described with respect to the use of organo titanates.

Formula II 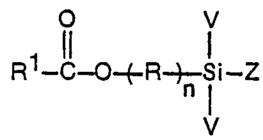

wherein $R^1$ is a hydrocarbon radical, as for example an alkyl radical having one to 18 carbon atoms inclusive, preferably one to four carbon atoms inclusive such as methyl, ethyl, *n*-propyl, isopropyl, and n-butyl; alkylene radicals having two to 18 carbon atoms inclusive, preferably two to 4 carbon atoms inclusive such as ethylene, and propylene; aryl radicals having six to ten carbon atoms inclusive such as phenyl, and benzyl. Other variable are as previously defined.

Exemplary of suitable silanes falling within the scope of Formula II are the following:

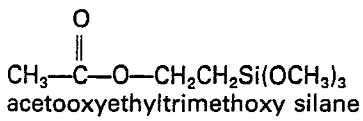
acetooxyethyltrimethoxy silane

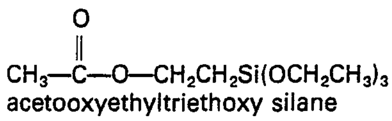
acetooxyethyltriethoxy silane

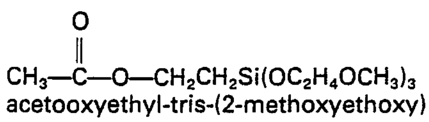
acetooxyethyl-tris-(2-methoxyethoxy) silane

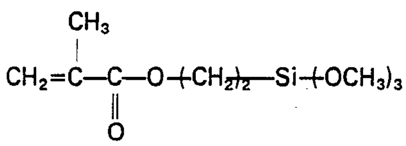
methacryloxyethyltrimethoxy silane

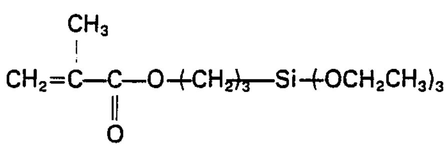
α-methylacryloxypropyltriethoxy silane

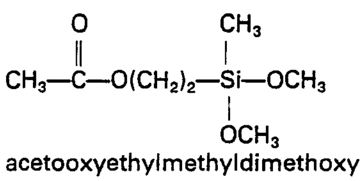
acetooxyethylmethyldimethoxy silane

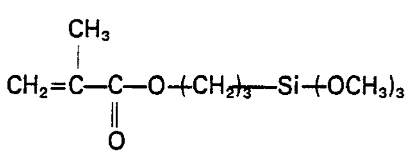
α-methacryloxypropyltrimethoxy silane

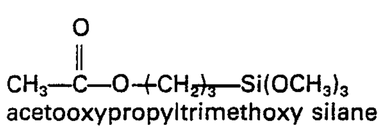
acetooxypropyltrimethoxy silane

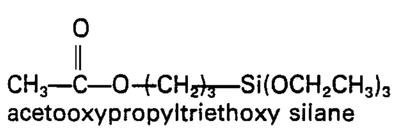
acetooxypropyltriethoxy silane

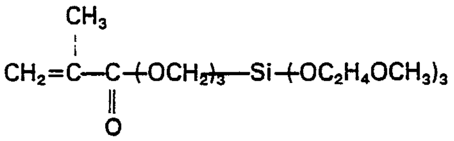

α-methacryloxypropyl-tris-(2-methoxyethoxy) silane

Preferred polysiloxanes, for purposes of this invention, contain repeating units falling within the scope of Formula I and have combined therein an organo titanate. The organo titanate modified polysiloxanes can be used as such, without the use of additional organo titanate catalyst, to react with the copolymers of alkylene-alkyl acrylate.

The preferred polysiloxanes have a viscosity of from 0.05 to 15 $Ns/m^2$ (0.5 to 150 poise), preferably from 0.1 to 2 $Ns/m^2$ (one to 20 poise) as determined by a Gardner-Holt bubble viscometer at a temperature of 25°C.

Organo titanate modified polysiloxanes can be prepared by reacting a mixture containing a silane falling within the scope of Formula II with an organo titanate falling within the scope of Formula III.

Formula III $$Ti(OR^2)_4$$

wherein each $R^2$, which can be the same or different, is hydrogen or a hydrocarbon radical having one to 18 carbon atoms inclusive, preferably one to 14 carbon atoms inclusive.

Exemplary of suitable hydrocarbon radicals are alkyl radicals such as methyl, ethyl, *n*-propyl, isopropyl, butyl, octyl, lauryl, myristyl, and stearyl, cycloaliphatic radicals such as cyclopentyl, and cyclohexyl, aryl radicals such as phenyl, methylphenyl, and chlorophenyl; alkaryl radicals such as benzyl.

Particularly desirable titanates falling within the scope of Formula III are those wherein each $R^2$ is alkyl having one to 18 carbon atoms inclusive, preferably one to 14 carbon atoms inclusive, exemplified by tetrabutyl titanate, and tetraisopropyl titanate.

Organo titanates falling within the scope of Formula III are known compounds and can be conveniently prepared as described in U.S.—A—2,984,641 to Leon E. Wolinski patented May 16, 1961.

Other suitable organo titanates are the organo titanium chelates such as tetraoctylene glycol titanium, triethanol amine titanate, titanium acetyl acetonate, and titanium lactate.

At least a catalytic amount of organo titanate is used to produce the organo titanate modified polysiloxanes, that is an amount sufficient to catalyze the condensation and polymerization reaction to produce a polysiloxane. Generally, the amount of organo titanate used is of the order of from 0.001 to 25 percent by weight based on the weight of the monomeric silane. It is preferred to use from 0.5 to 5 percent by weight of organo titanate based on the weight of the monomeric silane.

The temperature at which the reaction is conducted can be varied over a wide range, for example from 0°C to 250°C. A temperature in the range of from 70°C to 130°C is preferred. Also the reaction can be conducted using a suitable solvent, illustrated by hydrocarbon solvents such as toluene, xylene, cumene, decaline, dodecane, and chlorobenzene.

The reaction between the organo titanate and the monomeric silane can be conducted under atmospheric, subatmospheric or superatmospheric pressure. It is preferred to conduct the later stages of the reaction under subatmospheric pressure to allow for more facile removal of volatile by-products. Also, the reaction is preferably conducted under the atmosphere of an inert gas such as nitrogen or argon to avoid formation of a gel due to the water sensitivity of the product.

Control of the repeating unit, Formula I, of the polysiloxane can be effected by introducing an end blocker, as for example, a high boiling ester into the reaction mixture, at the beginning of the reaction or at any convenient point in the reaction process.

The number of repeating units of the polysiloxane is equal to the mole ratio of the monomeric silane to the end blocker as exemplified by the following simplified reaction scheme wherein the silane is shown to be acetooxyethyltrimethoxy silane and the high boiling ester is shown to be methyl benzoate.

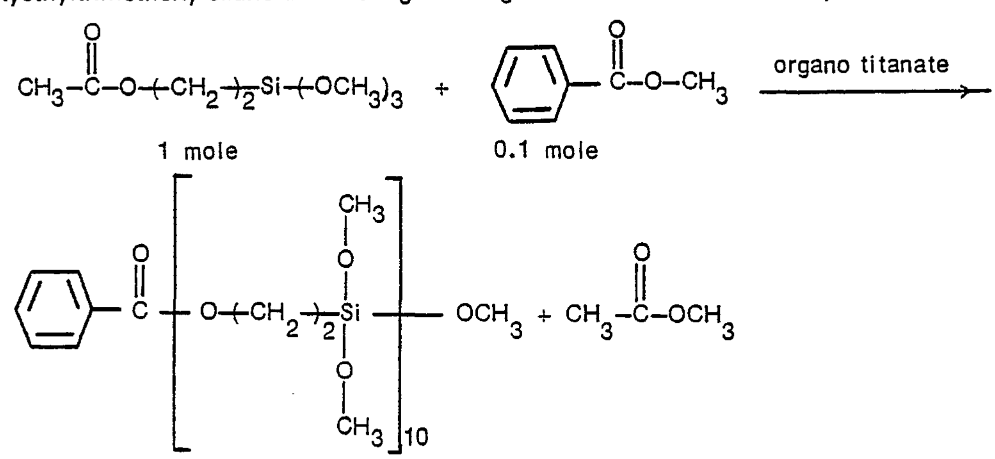

Suitable end blockers have the general formula

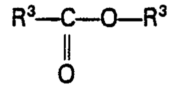

wherein each $R^3$, which can be the same or different is a hydrocarbon radical as defined for $R^2$.

Completion of the reaction is evidenced by cessation of the evolution of volatiles and the weight/volume of volatiles collected as compared to the theoretical weight/volume. Alternatively, the reaction can be run to a desired viscosity level and the reactants cooled to stop the reaction.

The alkylene-alkyl acrylate copolymers with which the polysiloxanes are reacted to form the silane modifed copolymers are known copolymers produced by reacting an alkene with an alkyl acrylate.

Suitable alkenes are ethylene, propylene, butene-1, isobutylene, pentene-1, 2-methylbutene-1, 3-methylbutene-1 hexene, heptene-1, octene-1, vinyl chloride, and styrene.

The alkylene moiety of the alkylene-alkyl acrylate copolymer generally contains from 2 to 18 carbon atoms inclusive, preferably 2 to 3 carbon atoms inclusive.

Suitable alkyl acrylate monomers which are copolymerized with the alkenes fall within the scope of the following formula:

Formula IV

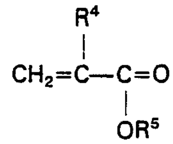

wherein $R^4$ is hydrogen or methyl and $R^5$ is alkyl having one to 8 carbon atoms inclusive. Illustrative compounds encompassed by this formula are: methyl acrylate, ethyl acrylate, t-butyl acrylate, methyl methacrylate, *n*-butylacrylate, n-butylmethacrylate, and 2-ethylhexyl acrylate.

Alkylene-alkyl acrylate copolymers generally have a density (ASTM D 1505) with conditioning as in ASTM D 147—72) of from 0.92 to 0.94 $kg/m^3$ and a melt index (ASTM-1238 of 303 kPa — (44 psi) tested pressure) of from 0.83 to 834 mg/S (0.5 to 500 decigrams per minute).

For purpose of the present invention, the preferred copolymer is a copolymer of ethylene-ethyl acrylate, generally having from one to 50 percent by weight combined ethyl acrylate, preferably having from 2 to 20 percent by weight combined ethyl acrylate.

The production of a silane modified copolymer of an alkylene-alkyl acrylate is carried out by reacting a polysiloxane, as described, with a copolymer of an alkylene-alkyl acrylate in the presence of an organo titanate catalyst.

In those instances wherein the polysiloxane contains combined organo titanate, additional organo titanate catalyst may not be necessary, especially when at least 0.5 percent by weight organo titanate, based on the weight of the monomeric silane, was used in the preparation of the polysiloxane.

The amount of organo titanate catalyst added to the reaction mixture is a catalytic amount, sufficent to catalyze the reaction between the polysiloxane and the copolymer. A preferred amount is from 0.001 to 50 percent by weight, most preferably from 0.1 to 25 percent by weight based on the weight of the polysiloxane.

The amount of polysiloxane used can vary from 0.05 to 10 and preferably from 0.3 to 5 percent by weight based on the weight of the copolymer.

The temperature at which this reaction is carried out is not critical and can vary, conveniently, from 80°C to 300°C and preferably from 150°C to 230°C.

The reaction can be carried out at atmospheric, subatmospheric or superatmospheric pressure, although atmospheric pressure is preferred and in the presence of solvents as previously described.

Completion of the reaction is evidenced by measurement of no further viscosity change.

Recovery of the silane modified copolymer is effected by allowing the contents of the reaction flask to cool and discharge to a suitable receiver for storage preferably under an inert gas blanket.

The reaction can be carried out in any suitable apparatus, preferably an apparatus in which the copolymer is subjected to mechanical working such as a Brabender mixer, a Banbury mixer or an extruder. The polysiloxane can be added to the fluxed copolymer and the organo titanate, if needed, then added. Alternatively, the organo titanate, if needed, can be added to the copolymer prior to the addition of the polysiloxane. Also, organo titanate and polysiloxane can be premixed and added to the fluxed polymer.

The reaction between the alkylene-alkyl acrylate copolymer and the polysiloxane may be depicted by the following equation:

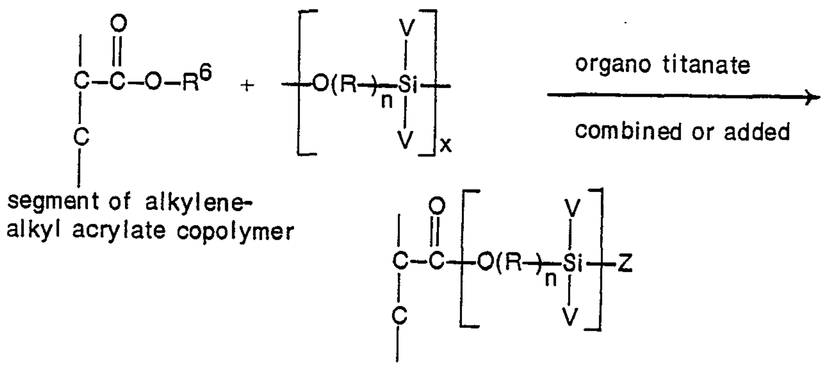


wherein the variables are as previously defined and the silicon containing unit is present in an amount of at least 0.05 percent by weight, generally from 0.1 to 10 percent by weight and preferably from 0.3 to 5 percent by weight based on the total weight of the modified copolymer.

The curing or crosslinking of the silane modified alkylene-alkyl acrylate copolymer is effected by exposing the copolymer to moisture. The moisture present in the atmosphere is usually sufficient to permit curing to occur over a period of 48 hours.

The rate of curing, in a matter of 30 minutes, can be accelerated by exposure to an artificially humidified atmosphere or immersion in water, and heating to elevated temperatures or by exposure to steam.

Generally, curing is effected at temperatures of the order of from 23°C to 100°C, preferably from 70°C to 100°C.

Additionally, the crosslinking may be carried out in the presence of a silanol condensation catalyst. A unique feature of this invention is that the crosslinking reaction can be carried out at significant rates in the absence of added silanol condensation catalyst. The organo titanate catalyst or catalyst residues present in the production of the silane modified copolymers also catalyze the crosslinking reaction.

Alternatively, a wide variety of materials which function as silanol condensation catalysts and which are known in the art can be employed in the crosslinking process. Such materials include metal carboxylates previously described; organic bases such as ethylamine, hexylamine dibutylamine and piperidine, and acids such as mineral acids and fatty acids.

To the silane modified copolymers of this invention may be added various additives, in amounts well known in the art, such as fillers among which can be mentioned carbon black, clay, talc, magnesium silicate, calcium carbonate, silica, and aluminum hydroxide.

The silane modified copolymers can be redered flame retardant by the addition thereto of halogen containing flame retardants such as decabromodiphenyl oxide, chlorinated polyethylene, polyvinyl chloride and halogenated paraffin waxes, alone, or in admixture with organic or inorganic antimony compounds such as antimony oxide and/or alkaline earth, metal oxides, carbonates, hydroxides and sulfates. Among such alkaline earth metal compounds can be noted calcium oxide, calcium carbonate, calcium hydroxide, calcium sulfate, magnesium oxide, magnesium carbonate, magnesium hydroxide and magnesium sulfate.

The following Examples further illustrate the present invention.

## Example 1

A. Preparation of Polysiloxane

The reaction scheme for the preparation of the polysiloxane can be depicted as follows wherein the silane monomer was acetooxyethyltrimethoxy silane:

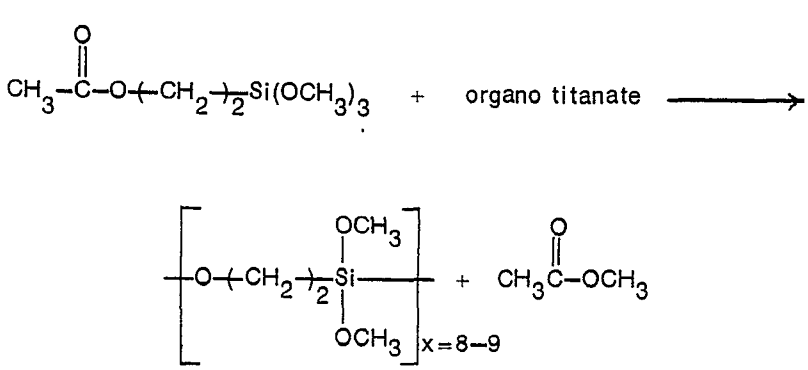

One hundred and four grams (0.5 mole) of acetooxyethyltrimethoxy silane were placed in a 250 ml. three-necked, round bottom flask and heated to a temperature of 75°C under a nitrogen gas atmosphere. When the contents of the flask reached a temperature of 75°C, 1.191 grams of tetraisopropyl titanate were added thereto using a syringe. The reaction mixture was heated for three hours at a temperature of 95°C—110°C. Volatiles evolved during the reaction and were condensed in a dry ice trap. At the end of the three hour period, the contents of the flask were cooled to room temperature, about 23°C, the residue removed from the flask, weighed and stored under argon.

| YIELD | ACTUAL | THEORETICAL | PERCENT OF THEORETICAL |
|---|---|---|---|
| Volatiles | 32.9 grams | 37 grams | 87 |
| Polysiloxane Product | 69.0 grams | 71.8 grams | 96 |

Viscosity of Polysiloxane Product — 0.34 $Ns/m^2$ (3.4 poise)

Infrared Analysis

| | |
|---|---|
| Volatiles | strong absorption at 1685 reciprocal centimeters which is consistent with strong adsorption at 1690 reciprocal centimeters for a known sample of methyl acetate. |
| Polysiloxane Product | strong absorption at 1080 reciprocal centimeters which is consistent with Si—O—$CH_3$ group; weak absorption at 1692 reciprocal centimeters which is consistent with significant reduction of carbonyl groups. |

Value of n as 8—9 is consistent with viscosity of product and amount of volatiles recovered.

B. Preparation of Silane Modified Copolymer of Ethylene-Ethyl Acrylate

To a 300 ml Brabender mixer heated to a temperature of 160°C and maintained under a blanket of argon gas, there was added 244 grams of a copolymer of ethylene-ethyl acrylate, having a melt index of 1.2 and containing 16 percent by weight combined ethyl acrylate and 1.26 grams of 1,2-dihydro-2,3,4-trimethyl quinoline, an antioxidant. The mixture was fluxed and mixed rapidly for 2 minutes. To the fluxed mixture, there was added, by means of a syringe, 5.80 grams of a mixture of the polysiloxane of (A) and dibutyltin dilaurate. The 5.80 gram mixture contained 97 percent by weight polysiloxane and 3 percent by weight dibutyltin dilaurate. After homogeneity was reached in the Brabender, as indicated by a constant torque measurement, 1.26 grams of tetraisopropyl titanate were added to the contents of the Brabender. The contents of the Brabender were then maintained at a temperature of 160—170°C for a period of 30 minutes resulting in a reaction whereby the silane reacted with the ethylene-ethyl acrylate copolymer evidenced by an increase in torque. Volatiles which evolved during the reaction were condensed in a dry ice trap which was connected to the Brabender. At the end of the 30 minute period, the contents of the Brabender were discharged into a polyethylene bag under an atmosphere of argon.

The volatiles which were collected weighed 0.03 grams.

Example 2

This example was conducted in the same manner as Example 1 with the exception that no additional tetraisopropyl titanate was used. The reactants and amounts thereof were as follows:

| | |
|---|---|
| Ethylene-ethyl acrylate copolymer (same as used in Example 1) | 246 grams |
| 1,2-dihydro-2,3,4-trimethyl quinoline | 1.26 grams |
| Polysiloxane (prepared as described in Example 1A) | 5.80 grams |

The volatiles which were collected weighed 0.03 grams.

Control

To a 300 ml Brabender mixer heated to a temperature of 160°C and maintained under a blanket of argon gas, there was added 241 grams of a copolymer and ethylene-ethyl acrylate having a melt index of 1.2 and containing 16 percent by weight combined ethyl acrylate and 1.26 grams of 1,2-dihydro-2,3,4-trimethyl quinoline. This mixture was fluxed and mixed rapidly for 2 minutes. To the fluxed mixture, there

was added, by means of a syringe, 8.84 grams of a mixture of acetooxyethyltrimethoxy silane

$$(CH_3-\underset{\underset{O}{\|}}{C}-O-(CH_2)_2\,Si(OCH_3)_3$$

and dibutyltin dilaurate. The 8.84 gram mixture contained 97 percent by weight acetooxyethyltrimethoxy silane and 3 percent by weight dibutyltin dilaurate. After homogeniety was reached in the Brabender, as indicated by a constant torque measurement, 1.26 grams of tetraisopropyl titanate were added to the contents of the Brabender. The contents of the Brabender were then maintained at a temperature of 160—170°C for a period of thirty minutes resulting in a reaction whereby the silane reacted with the ethylene-ethyl acrylate copolymer. Volatiles which evolved during the reaction were condensed in a dry ice trap which was connected to the Brabender. At the end of the thirty minute period, the contents of the Brabender were discharged into a polyethylene bag under an atmosphere of argon.

The volatiles which were collected weighed 3.01 grams.

20 gram samples of silane modified copolymers of Example 1, Example 2 and Control 1 were pressed into plaques of the following dimensions: 76.2 mm × 76.2 mm × 1.91 mm (3 inches × 3 inches × .075 inch) in a five minute cycle at a temperature of 110°C—115°C under a pressure of 34.5 MPa (5000 psig).

The plaques were cured by being suspended in water, which was at a temperature of 90°C, for three hours. After the three hour water-cure, the plaques were removed from the water, wiped dry and placed in a vacuum oven, which was at a temperature of 50°C, for one hour in order to ensure removal of residual water.

The plaques were then measured for degree of cross-linking, according to the Monsanto Rheometer test. This test procedure is described, more fully, in U.S.—A—4,018,852 to Donald L. Schober, granted April 19, 1977. Figure 1 of the drawing of this patent shows the typical Rheometer curve. The level of vulcanization or crosslinking is designated as H and is measured in terms of m-kg (inch-pounds) of torque on the Rheometer test equipment.

Results with respect to the plaques tested are as follows:

| | Average of (2) Plaques Tested |
|---|---|
| Plaques of Example 1 | 0.61 m-kg (53 inch-pounds) |
| Plaques of Example 2 | 0.61 m-kg (53 inch-pounds) |
| Plaques of Control 1 | 0.61 m-kg (53 inch-pounds) |

Results show that a copolymer reacted with a polysiloxane has the same degree of crosslinking, with evolution of significantly less volatiles, than a copolymer reacted with a monomeric silane.

## Example 3

This example illustrates the preparation of a polysiloxane using an end blocker.

A three liter flask was charged with 2140 grams and 10.29 moles of acetooxyethyltrimethoxy silane, 154 grams (1.03 moles) of ethyl benzoate and the contents of the flask brought to a temperature of 85°C. To this mixture, there were then added 21 grams of tetraisopropyl titanate. The solution, kept under an argon gas atmosphere, was stirred while being heated for 5¾ hours at a temperature of 94°C—125°C. During this period of time, 752 grams of volatiles were collected in an acetone dry ice trap. This was 98.8 percent of the theoretical amount of methyl acetate based on 100 percent conversion. The polysiloxane product recovered weighed 1543 grams, 99.3 percent of the theoretical yield. Viscosity of the product was 0.14 Ns/$m^2$ (1.4 poise). The equation for the preparation is as follows:

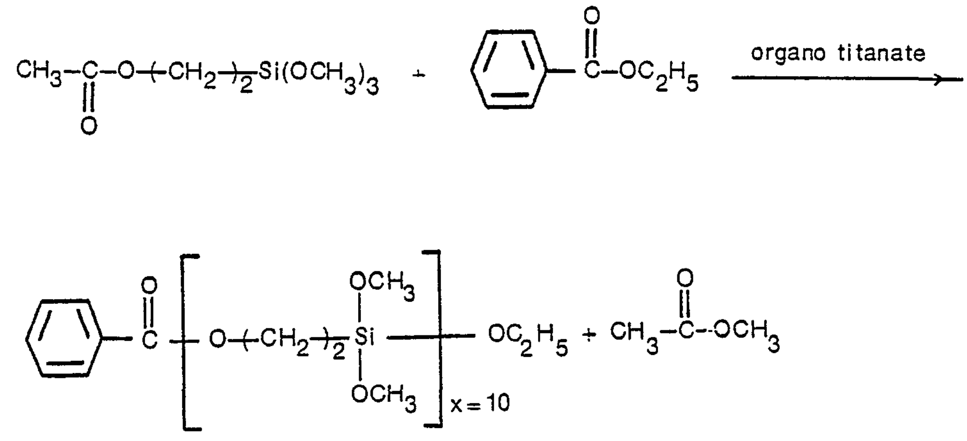


## Example 4

Example 3 was repeated using the same reactants, same molar amounts and same reaction conditions with the exception that acetooxyethylmethyldimethoxy silane was used as the monomeric silane. Viscosity of the product was 0.14 $Ns/m^2$ (1.4 poise).

The silane modified copolymers of alkylene-alkyl acrylates, although described primarily for use as insulation can also be used as jacketing about industrial cables telephone wires and cables.

**Claims**

1. A process of producing a water-curable, silane-modified alkylene-alkyl acrylate copolymer which comprises reacting a mixture containing an alkylene-alkyl acrylate copolymer; a polysiloxane having the repeating unit:

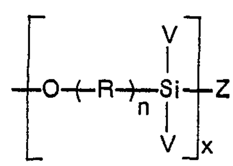

wherein R is a hydrocarbon radical, each V is hydrogen, a hydrocarbon radical or hydrolyzable group and Z is a hydrolyzable group, n is an integer having a value of 1 to 18 inclusive, and x is an integer having a value of at least 2; and an organic titanate.

2. A process as claimed in claim 1 when conducted at a temperature of from 80°C to 300°C.

3. A process as claimed in claim 2 when conducted at a temperature of from 150°C to 230°C.

4. A process as claimed in any one of the preceding claims wherein R is an alkylene radical having 1 to 18 carbon atoms inclusive.

5. A process as claimed in any one of the preceding claims wherein each V is an alkoxy radical having 1 to 18 carbon atoms inclusive.

6. A process as claimed in any one of the preceding claims wherein Z is an alkoxy radical having 1 to 18 carbon atoms.

7. A process as claimed in any one of the preceding claims wherein R is $—CH_2CH_2$, and V and Z are methoxy.

8. A process as claimed in any one of claims 1 to 6 wherein R is $—CH_2CH_2—$, Z is methoxy, and V is methoxy and dodecyloxy.

9. A process as claimed in any one of claims 1 to 6 wherein R is $—CH_2CH_2—$, Z is methoxy, and V is alkyl and methoxy.

10. A process as claimed in any one of the preceding claims wherein x has a value of 5 to 25 inclusive.

11. A process as claimed in any one of the preceding claims wherein the organo titanate has the formula:

$$Ti(OR^2)_4$$

wherein each $R^2$ is hydrogen or a hydrocarbon radical having from one to 18 carbon atoms inclusive.

12. A process as claimed in claim 11 wherein each $R^2$ is an alkyl radical having 1 to 14 carbon atoms inclusive.

13. A process as claimed in claim 12 wherein the organo titanate is tetraisopropyl titanate or tetrabutyl titanate.

14. A process as claimed in any one of the preceding claims wherein the organo titanate is present in an amount of from 0.1 to 25 percent by weight based on the weight of the polysiloxane.

15. A process as claimed in any one of the preceding claims wherein the polysiloxane is present in an amount of from 0.05 to 10 percent by weight based on the weight of the copolymer.

16. A process as claimed in any one of the preceding claims wherien the said copolymer is a copolymer of ethylene-ethyl acrylate.

17. A process as claimed in any one of the preceding claims wherein the polysiloxane contains an organo titanate chemically combined therewith.

18. A polysiloxane reactive with an alkylene-alkyl acrylate copolymer and containing the repeating unit:

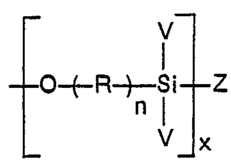

wherein R is a hydrocarbon radical, each V is hydrogen, a hydrocarbon radical or a hydrolyzable group, Z is a hydrolyzable group, n is an integer having a value of 1 to 18 inclusive and x is an integer having a value of at least 2, said polysiloxane being end-blocked by an ester group and containing an organo titanate chemically combined therewith.

19. A polysiloxane as claimed in claim 18 wherein R is an alkylene radical having 1 to 18 carbon atoms inclusive.

20. A polysiloxane as claimed in claim 18 or claim 19 wherein each V is an alkoxy radical having 1 to 18 carbon atoms inclusive.

21. A polysiloxane as claimed in any one of the claims 18 to 20 wherein Z is an alkoxy radical having 1 to 18 carbon atoms inclusive.

22. A polysiloxane as claimed in claim 21 wherein R is $—CH_2CH_2—$, and V and Z are methoxy.

23. A polysiloxane as claimed in claim 21 wherein R is $—CH_2CH_2—$, Z is methoxy, V is methoxy and dodecyloxy.

24. A polysiloxane as claimed in claim 21 wherein R is $—CH_2CH_2—$, Z is methoxy and V is alkyl and methoxy.

25. A polysiloxane as claimed in any one of claims 18 to 24 wherein the organo titanate has the formula:

$$Ti(OR^2)_4$$

wherein each $R^2$ is hydrogen or a hydrocarbon radical having from one to 18 carbon atoms inclusive.

26. A polysiloxane as claimed in claim 25 wherein $R^2$ is an alkyl radical having 1 to 14 carbon atoms inclusive.

27. A polysiloxane as claimed in claim 26 wherein the organo titanate is tetraisopropyl titanate or tetrabutyl titanate.

28. A polysiloxane as claimed in any one of claims 18 to 27 wherein x has a value of 5 to 25 inclusive.

29. A polysiloxane as claimed in any one of claims 18 to 28 wherein the end blocking group has the formula:

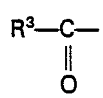

wherein $R^3$ is a hydrocarbon radical having from one to 18 carbon atoms inclusive.

30. A polysiloxane as claimed in claim 29 wherein the end blocking group is benzoate.

31. A polysiloxane as claimed in claim 30 wherein the organo titanate is tetraisopropyl titanate and the polysiloxane is derived from acetooxyethyltrimethoxy silane or acetoxyethylmethyldimethoxy silane.

32. A process for producing a polysiloxane as claimed in claim 18, which process comprises reacting a mixture consisting essentially of an organo titanate and a silane of the formula:

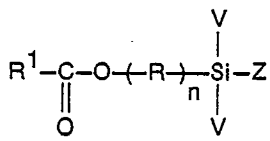

wherein R is a hydrocarbon radical, $R^1$ is a hydrocarbon radical, each V is hydrogen, a hydrocarbon radical or a hydrolyzable group, Z is a hydrolyzable group, and n is an integer having a value of 1 to 18 inclusive.

33. A process as claimed in claim 32 wherein the organo titanate is present in an amount of from 0.001 to 25 percent by weight, based on the weight of the said silane.

34. A process as claimed in claim 32 or claim 33 when conducted at a temperature of from 0°C to 250°C.

35. A process as claimed in any one of claims 32 to 34 wherein the organo titanate has the formula:

$$Ti(OR^2)_4$$

wherein each $R^2$ is hydrogen or a hydrocarbon radical having from one to 18 carbon atoms inclusive.

36. A process as claimed in claim 35 wherein $R^2$ is an alkyl radical having 1 to 14 carbon atoms inclusive.

37. A process as claimed in claim 36 wherein the organo titanate is tetraisopropyl titanate or tetrabutyl titanate.

38. A process as claimed in any one of claims 32 to 37 wherein said mixture contains and end blocker.

39. A process as claimed in claim 38 wherein the end blocker has the formula:

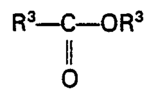

wherein each $R^3$ is a hydrocarbon radical having from one to 18 carbon atoms inclusive.

40. A process as claimed in claim 39 wherein the end blocker is ethyl benzoate or methyl benzoate.

41. A process as claimed in claim 40 wherein the organo titanate is tetraisopropyl titanate and the silane is acetooxyethyltrimethoxy silane or acetooxyethylmethyldimethoxy silane.

42. An electrical conductor, such as a wire or cable, having an insulation formed by water-curing a water-curable silane-modified alkylene-alkyl acrylate copolymer produced by a process as claimed in any one of claims 1 to 17.

**Patentansprüche**

1. Verfahren zur Herstellung eines wasserhärtbaren silanmodifizierten Alkylen-Alkylacrylat-Copolymerisates, dadurch gekennzeichnet, daß man eine Mischung, die ein Alkylen-Alkylacrylat-Copolymerisat, ein Polysiloxan mit der wiederkehrenden Einheit

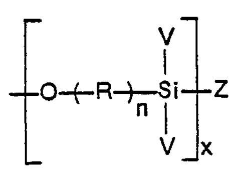

worin R ein Kohlenwasserstoffrest ist,
jedes V Wasserstoff, eine Kohlenwasserstoffrest oder eine hydrolysierbare Gruppe ist, und Z eine hydrolysierbare Gruppe ist, n eine ganze Zahl mit einem Wert von 1 bis 18 einschließlich und x ein ganze Zahl mit einem Wert von mindestens 2 ist, und ein organisches Titanat enthält, umsetzt.

2. Verfahren nach Anspruch 1, wenn es bei einer Temperatur von 80 bis 300°C durchgeführt wird.

3. Verfahren nach Anspruch 2, wenn es bei einer Temperatur von 150 bis 230°C durchgeführt wird.

4. Verfahren nach einem der vorhergehenden Ansprüche, in welchem R ein Alkylenrest mit 1 bis 18 Kohlenstoffatomen einschließlich ist.

5. Verfahren nach einem der vorhergehenden Ansprüche, in welchem jedes V ein Alkoxyrest mit 1 bis 18 Kohlenstoffatomen einschließlich ist.

6. Verfahren nach einem der vorhergehenden Ansprüche, in welchem Z ein Alkoxyrest mit 1 bis 18 Kohlenstoffatomen ist.

7. Verfahren nach einem der vorhergehenden Ansprüche, in welchem R $-CH_2CH_2-$ ist und V und Z Methoxy bedeuten.

8. Verfahren nach einem der Ansprüche 1 bis 6, in welchem R $—CH_2CH_2—$ ist, Z Methoxy ist und V Methoxy und Dodecyloxy ist.

9. Verfahren nach einem der Ansprüche 1 bis 6, n welchem R $—CH_2CH_2—$ ist, Z Methoxy ist und V Alkyl und Methoxy ist.

10. Verfahren nach einem der vorhergehenden Ansprüche, in welchem x einen Wert von 5 bis 25 einschließlich hat.

11. Verfahren nach einem der vorhergehenden Ansprüche, in welchem das Organotitanat die Formel

$$Ti(OR^2)_4$$

hat, worin jedes $R^2$ Wasserstoff oder ein Kohlenwasserstoffrest mit 1 bis 18 Kohlenstoffatomen einschließlich ist.

12. Verfahren nach Anspruch 11, in welchem jedes $R^2$ ein Alkylrest mit 1 bis 14 Kohlenstoffatomen einschließlich ist.

13. Verfahren nach Anspruch 12, in welchem das Organotitanat Tetraisopropyltitanat oder Tetrabutyltitanat ist.

14. Verfahren nach einem der vorhergehenden Ansprüche, in welchem das Organotitanat in einer Menge von 0,1 bis 25 Gew.-%, bezogen auf das Gewicht des Polysiloxans, anwesend ist.

15. Verfahren nach einem der vorhergehenden Ansprüche, in welchem das Polysiloxan in einer Menge von 0,05 bis 10 Gew.-%, bezogen auf das Gewicht des Copolymerisates, anwesend ist.

16. Verfahren nach einem der vorhergehenden Ansprüche, in welchem das Copolymerisat ein Copolymerisat aus Ethylen-Ethylacrylat ist.

17. Verfahren nach einem der vorhergehenden Ansprüche, in welchem das Polysiloxan ein chemisch damit kombiniertes Organotitanat enthält.

18. Ein mit einem Alkylen-Alkylacrylat-Copolymerisat reaktionsfähiges Polysiloxan, das die wiederkehrende Einheit

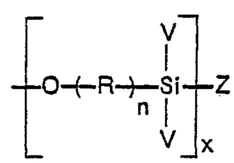

enthält, worin R ein Kohlenwasserstoffrest ist, jedes V Wasserstoff, ein Kohlenwasserstoffrest oder eine hydrolysierbare Gruppe ist, z eine hydrolysierbare Gruppe ist, n eine ganze Zahl mit einem Wert von 1 bis 18 einschließlich ist und z ein ganze Zahl mit einem Wert von mindestens 2 ist, wobei das Polysiloxan durch eine Estergruppe endblockiert ist und ein chemisch damit kombiniertes Organotitanat enthält.

19. Polysiloxan nach Anspruch 18, in welchem R ein Alkylrest mit 1 bis 18 Kohlenstoffatomen einschließlich ist.

20. Polysiloxan nach Anspruch 18 oder 19, in welchem jedes V ein Alkoxyrest mit 1 bis 18 Kohlenstoffatomen einschließlich ist.

21. Polysiloxan nach einem der Ansprüche 18 bis 20, in welchem Z ein Alkoxyrest mit 1 bis 18 Kohlenstoffatomen einschließlich ist.

22. Polysiloxan nach Anspruch 21, in welchem R $—CH_2CH_2—$ ist und V und Z Methoxy sind.

23. Polysiloxan nach Anspruch 21, in welchem R $—CH_2CH_2—$ ist, Z Methoxy ist und V Methoxy und Dodecyloxy ist.

24. Polysiloxan nach Anspruch 21, in welchem R $—CH_2CH_2—$ ist, Z Methoxy ist und v Alkyl und Methoxy ist.

25. Polysiloxan nach einem der Ansprüche 18 bis 24, in welchem das Organotitanat die Formel

$$Ti(OR^2)_4$$

hat, worin jedes $R^2$ Wasserstoff oder ein Kohlenwasserstoffrest mit 1 bis 18 Kohlenstoffatomen einschließlich ist.

26. Polysiloxan nach Anspruch 25, in welchem $R^2$ ein Alkylrest mit 1 bis 14 Kohlenstoffatomen einschließlich ist.

27. Polysiloxan nach Anspruch 26, in welchem das Organotitanat Tetrabutyltitanat ist.

28. Polysiloxan nach einem der Ansprüche 18 bis 27, in welchem x eine Wert von 5 bis 25 einschließlich hat.

29. Polysiloxan nach einem der Ansprüche 18 bis 28, in welchem die endblockierende Gruppe die Formel

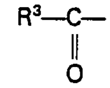

hat, worin $R^3$ ein Kohlenwasserstoffrest mit 1 bis 18 Kohlenstoffatomen einschließlich ist.

30. Polysiloxan nach Anspruch 29, in welchem die endblockierende Gruppe Benzoat ist.

31. Polysiloxan nach Anspruch 30, in welchem das Organotitanat Tetraisopropyltitanat ist und das Polysiloxan von Acetoxyethyltrimethoxysilan oder Acetoxyethylmethyldimethoxysilan hergeleitet ist.

32. Verfahren zur Herstellung eines Polysiloxans nach Anspruch 18, dadurch gekennzeichnet, daß man eine Mischung, die im wasentlichen aus einem Organotitanat und einem Silan der Formel

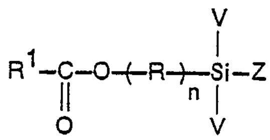

besteht, worin R ein Kohlenwasserstoffrest ist, $R^1$ ein Kohlenwasserstoffrest ist, jedes V Wasserstoff, ein Kohlenwasserstoffrest oder eine hydrolysierbare Gruppe ist, Z eine hydrolysierbare Gruppe ist und n eine ganze Zahl mit einem Wert von 1 bis 18 einschließlich ist, umsetzt.

33. Verfahren nach Anspruch 32, in welchem das Organotitanat in einer Menge von 0.001 bis 25 Gew.-%, bezogen auf das Gewicht des Silans, anwesend ist.

34. Verfahren nach Anspruch 32 oder 33, wenn es bei einer Temperatur von 0 bis 250°C durchgeführt wird.

35. Verfahren nach einem der Ansprüche 32 bis 34, worin das Organotitanat die Formel

$$Ti(OR^2)_4$$

hat, worin jedes $R^2$ Wasserstoff oder ein Kohlenwasserstoffrest mit 1 bis 18 Kohlenstoffatomen einschließlich ist.

36. Verfahren nach Anspruch 35, worin $R^2$ ein Alkylrest mit 1 bis 14 Kohlenstoffatomen einschließlich ist.

37. Verfahren nach Anspruch 36, in welchem das Organotitanat Tetraisopropyltitanat oder Tetrabutyltitanat ist.

38. Verfahren nach einem der Ansprüche 32 bis 37, in welchem die Mischung eine Endblockierungsverbindung enthält.

39. Verfahren nach Anspruch 38, in welchem die Endblockierungverbindung die Formel

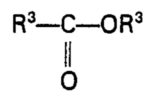

hat, worin jedes $R^3$ ein Kohlenwasserstoffrest mit 1 bis 18 Kohlenstoffatomen einschließlich ist.

40. Verfahren nach Anspruch 39, in welchem die Endblockierungsverbindung Ethylbenzoat oder Methylbenzoat ist.

41. Verfahren nach Anspruch 40, in welchem das Organotitanat Tetraisopropyltitanat ist und das Silan Acetoxyethyltrimethoxysilan oder Acetoxyethylmethyldimethoxysilan ist.

42. Elektrischer Leiter, z.B. ein Draht oder Kabel, mit einer Isolierung, die durch ein wasserhärtbares silanmodifiziertes Alkylen-Alkylacrylat-Copolymerisat gebildet wird, das nach einem Verfahren gemäß einem der Ansprüche 1 bis 17 hergestellt ist.

**Revendications**

1. Procédé de production d'un copolymère alkylène-acrylate d'alkyle modifié par un silane, réticulable par l'eau, qui consiste à faire réagir un mélange contenant un copolymère alkylène-acrylate d'alkyle, un polysiloxane portant le motif répété:

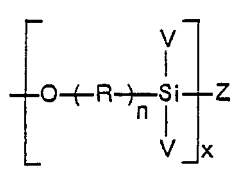

dans lequel R est un radical hydrocarboné,

chaque V représente l'hydrogène, un radical hydrocarboné ou un groupe hydrolysable et Z est un groupe hydrolysable, n est un nombre entier ayant une valeur de 1 à 18 inclus, et x est un nombre entier ayant au moins la valeur 2; et un titanate organique.

2. Procédé suivant la revendication 1, mis en oeuvre à une température de 80 à 300°C.

3. Procédé suivant la revendication 2, mis en oeuvre à une température de 150 à 230°C.

4. Procédé suivant l'une quelconque des revendications précédentes, dans lequel R est un radical alkylène ayant 1 à 18 atomes de carbone inclus.

5. Procédé suivant l'une quelconque des revendications précédentes, dans lequel chaque V représente un radical alkoxy ayant 1 à 18 atomes de carbone inclus.

6. Procédé suivant l'une quelconque des revendications précédentes, dans lequel Z est un radical alkoxy ayant 1 à 18 atomes de carbone.

7. Procédé suivant l'une quelconque des revendications précédentes, dans lequel R représente $—CH_2CH_2$, et V et Z sont des groupes méthoxy.

8. Procédé suivant l'une quelconque des revendications 1 à 6, dans lequel R est un groupe $—CH_2CH_2—$, Z est un groupe méthoxy et V représente des groupe méthoxy et dodécyloxy.

9. Procédé suivant l'une quelconque des revendications 1 à 6, dans lequel R est un groupe $—CH_2CH_2—$, Z est un groupe méthoxy, et V représente des groupes alkyle et méthoxy.

10. Procédé suivant l'une quelconque des revendications précédentes, dans lequel x a une valeur de 5 à 25 inclus.

11. Procédé suivant l'une quelconque des revendications précédentes, dans lequel le titanate organique répond à la formule:

$$Ti(OR^2)_4$$

dans laquelle chaque $R^2$ est l'hydrogène ou un radical hydrocarboné ayant 1 à 18 atomes de carbone inclus.

12. Procédé suivant la revendication 11, dans lequel chaque $R^2$ est un radical alkyle ayant 1 à 14 atomes de carbone inclus.

13. Procédé suivant la revendication 12, dans lequel le titanate organique est le titanate de tétraisopropyle ou le titanate de tétrabutyle.

14. Procédé suivant l'une quelconque des revendications précédentes, dans lequel le titanate organique est présent en une quantité de 0,1 à 25% en poids sur la base du poids du polysiloxane.

15. Procédé suivant l'une quelconque des revendications précédentes, dans lequel le polysiloxane est présent en une quantité de 0,05 à 10% en poids sur la base du poids du copolymère.

16. Procédé suivant l'une quelconque des revendications précédentes, dans lequel ledit copolymère est un copolymère d'éthylène et d'acrylate d'éthyle.

17. Procédé suivant l'une quelconque des revendications précédentes, dans lequel le polysiloxane contient un titanate organique en combinaison chimique avec lui.

18. Polysiloxane apte à réagir avec un copolymère alkylène-acrylate d'alkyle et contenant le motif répété:

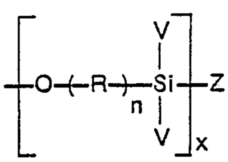

dans lequel R est un radical hydrocarboné,
chaque V représente l'hydrogène, un radical hydrocarboné ou un groupe hydrolysable, Z est un groupe hydrolysable, n est un nombre entier ayant une valeur de 1 à 18 inclus et x est un nombre entier ayant au moins la valeur 2, ledit polysiloxane étant terminé par un groupe ester et contenant un titanate organique en combinaison chimique avec lui.

19. Polysiloxane suivant la revendication 18, dans lequel R est un radical alkylène ayant 1 à 18 atomes de carbone inclus.

20. Polysiloxane suivant la revendication 18 ou la revendication 19, dans lequel chaque V est un radical alkoxy ayant 1 à 18 atomes de carbone inclus.

21. Polysiloxane suivant l'une quelconque des revendications 18 à 20, dans lequel Z est un radical alkoxy ayant 1 à 18 atomes de carbone inclus.

22. Polysiloxane suivant la revendication 21, dans lequel R est un groupe $—CH_2CH_2—$, et V et Z sont des groupes méthoxy.

23. Polysiloxane suivant la revendication 21, dans lequel R est un groupe $—CH_2CH_2—$, Z est un groupe méthoxy et V est un groupe méthoxy ou dodécyloxy.

24. Polysiloxane suivant la revendication 21, dans lequel R est un groupe $—CH_2CH_2—$, Z est un groupe méthoxy et V est un groupe alkyle ou méthoxy.

25. Polysiloxane suivant l'une quelconque des revendications 18 à 24, dans lequel le titanate organique répond à la formule

$$Ti(OR^2)_4$$

dans laquelle chaque $R^2$ est l'hydrogène ou un radical hydrocarboné ayant 1 à 18 atomes de carbone inclus.

26. Polysiloxane suivant la revendication 25, dans lequel $R^2$ est un radical alkyle ayant 1 à 14 atomes de carbone inclus.

27. Polysiloxane suivant la revendication 26, dans lequel le titanate organique est le titanate de tétraisopropyle ou le titanate de tétrabutyle.

28. Polysiloxane suivant l'une quelconque des revendications 18 à 27, dans lequel x a une valeur de 5 à 25 inclus.

29. Polysiloxane suivant l'une quelconque des revendications 18 à 28, dans lequel le groupe de terminaison répond à la formule:

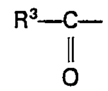

dans laquelle $R^3$ est un radical hydrocarboné 1 à 18 atomes de carbone inclus.

30. Polysiloxane suivant la revendication 29, dans lequel le groupe de terminaison est un groupe benzoate.

31. Polysiloxane suivant la revendication 30, dans lequel le titanate organique est le titanate de tétraisopropyle et le polysiloxane est dérivé de l'acétoxyéthyltriméthoxysilane ou de l'acétoxyéthylméthyldiméthoxysilane.

32. Procédé de production d'un polysiloxane suivant la revendication 18, procédé qui consiste à faire réagir un mélange essentiellement formé d'un titanate organique et d'un silane de formule

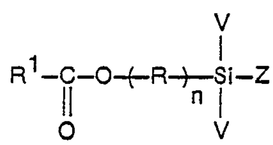

dans laquelle R est un radical hydrocarboné,
$R^1$ est un radical hydrocarboné, chaque V
représente l'hydrogène, un radical hydrocarboné ou un groupe hydrolysable, Z est un groupe hydrolysable et n est un nombre entier ayant une valeur de 1 à 18 inclus.

33. Procédé suivant la revendication 32, dans lequel le titanate organique est présent en une quantité de 0,001 à 25% en poids, sur la base du poids dudit silane.

34. Procédé suivant la revendication 32 ou la revendication 33, mis en oeuvre à une température de 0 à 250°C.

35. Procédé suivant l'une quelconque des revendications 32 à 34, dans lequel le titanate organique répond à la formule:

$$Ti(OR^2)_4$$

dans laquelle chaque $R^2$ est l'hydrogène ou un radical hydrocarboné ayant 1 à 18 atomes de carbone inclus.

36. Procédé suivant la revendication 35, dans lequel $R^2$ est un radical alkyle ayant 1 à 14 atomes de carbone inclus.

37. Procédé suivant la revendication 36, dans lequel le titanate organique est le titanate de tétraisopropyle ou le titanate de tétrabutyle.

38. Procédé suivant l'une quelconque des revendications 32 à 37, dans lequel ledit mélange contient un corps de blocage terminal.

39. Procédé suivant la revendication 38, dans lequel le corps de blocage terminal répond à la formule:

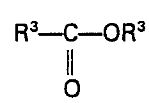

dans laquelle chaque $R^3$ est un radical hydrocarboné ayant 1 à 18 atomes de carbone inclus.

40. Procédé suivant la revendication 39, dans lequel le corps de blocage terminal est le benzoate d'éthyle ou le benzoate de méthyle.

41. Procédé suivant la revendication 40, dans lequel le titanate organique est le titanate de tétraisopropyle et le silane est l'acétoxyéthyltriméthoxysilane ou l'acétoxyéthylméthyldiméthoxysilane.

42. Conducteur électrique tel qu'un fil ou un câble portant un isolant formé par réticulation à l'eau d'un copolymère alkylène-acrylate d'alkyle modifié par un silane réticulable à l'eau, produit par un procédé suivant l'une quelconque des revendications 1 à 17.